# EUROPEAN PATENT SPECIFICATION

(11) **EP 3 003 474 B1**
(45) Date of publication and mention of the grant of the patent: **01.01.2020**
(21) Application number: 14735060.7
(22) Date of filing: 06.06.2014
(51) Int. Cl.: A61N 1/372

(54) **METHODS AND SYSTEMS FOR AUTHORIZING A PORTABLE DEVICE TO COMMUNICATE WITH A MEDICAL DEVICE**
VERFAHREN UND SYSTEME ZUR AUTORISIERUNG EINER TRAGBAREN VORRICHTUNG ZUR KOMMUNIKATION MIT EINER MEDIZINISCHEN VORRICHTUNG
PROCÉDÉS ET SYSTÈMES D'AUTORISATION DE COMMUNICATION D'UN DISPOSITIF PORTABLE AVEC UN DISPOSITIF MÉDICAL

(30) Priority: 06.06.2013 US 201361832076 P
(43) Date of publication of application: 13.04.2016
(73) Proprietor: Boston Scientific Neuromodulation Corporation, Valencia, CA 91355 (US)
(72) Inventor: HUERTO, Archie Eslabon, Simi Valley, California 93065 (US); CARBUNARU, Rafael, Valley Village, California 91607 (US); KOTHANDARAMAN, Sridhar, Valencia, California 91354 (US); ZOTTOLA, Dennis, Ventura, California 93003 (US)
(74) Representative: Vossius & Partner Patentanwälte Rechtsanwälte mbB
(86) International application number: PCT/US2014/041424
(87) International publication number: WO 2014/197875

(56) References cited:
- US-A1- 2005 283 198
- US-A1- 2010 292 556
- US-A1- 2012 163 663
- Medtronic: "MEDTRONIC CARELINK PROGRAMMER Model 2090 Programmer/Model 2290 Analyzer for Medtronic and Vitatron Devices Programmer Reference Guide" In: "MEDTRONIC CARELINK PROGRAMMER Model 2090 Programmer/Model 2290 Analyzer for Medtronic and Vitatron Devices Programmer Reference Guide", 1 January 2005 (2005-01-01), XP055523079,

## Description

### FIELD OF THE INVENTION

The present invention generally relates to the field of medical devices, and more particularly, to methods and systems for authorizing a portable device to communicate with an implantable medical device.

### BACKGROUND OF THE INVENTION

Generally, patient programmers or remote controllers (RCs) are provided to patients implanted with a medical device, such as a neurostimulator, to communicate with the implanted neurostimulator. For example, the programmer remotely instructs the neurostimulator to generate electrical stimulation pulses in accordance with selected stimulation parameters, or adjusts the neurostimulator output to obtain the best therapy. The programmer can also be used to increase or decrease the strength of stimulation, to select different areas of the body to be stimulated, to change therapy settings, and/or to activate or deactivate stimulation. However, the development of patient programmers may involve a substantial amount of investment and effort in terms of both time and expense. For example, development involves efforts to make the user interface easy to use. Like many electronic devices, the programmer includes a display, buttons, speakers, software for controlling the stimulator, and/or other components, which make it expensive and sometimes difficult for all patients to afford. Patients also may need to spend time, and sometimes a significant amount of time, attempting to understand how to use the programmer, and simultaneously the clinician or medical practitioner may need to spend a lot of effort to ensure that the patient understands the functionalities of the programmer. Moreover, the patient is also required to constantly carry this additional device.

Many users currently carry personal smart devices that offer advanced functions, such as email capabilities, internet browsing, multi-media content management, online shopping, etc. The personal smart devices provide convenience and functionality to users at any location and time. Due to the ubiquity of personal smart devices, it would be desirable to utilize these devices to control implantable medical devices. However, not all personal smart devices are capable of effectively and safely communicating with a particular implantable medical device.

There, thus, remains a need for such systems and methods for ensuring that a particular personal smart device can effectively and safely communicate with a particular implantable medical device.

### SUMMARY OF THE INVENTION

The invention is defined by claims 1 and 12. Preferred embodiments are defined by the dependent claims. Further embodiments disclosed herein are for exemplary purpose only.

In accordance with a first aspect of the present inventions, a method for authorizing a portable device (e.g., a personal smart device) to communicate with an implantable medical device (e.g., an implantable pulse generator (IPG)). The method comprises performing a test that determines whether at least one device configuration parameter respectively corresponding to at least one feature to be tested on the portable device is fulfilled by the portable device.

The feature to be tested on the portable device may, e.g., be one of a storage memory of the portable device, a random access memory of the portable device, a capability of the portable device to connect to a type of a network, a screen height of the portable device, a screen width of the portable device, an operating system of the portable device, a model of the portable device, a type of central processing unit of the portable device, a speed of central processing unit of the portable device, a type of graphic library of the portable device, a USB capability of the portable device, an API level of the portable device, a near field communication capability of the portable device, a Bluetooth® capability of the portable device, a wireless capability of the portable device, a hardware connectivity performance of the portable device, a type of security capability of the portable device, a sensitivity of a touchscreen of the portable device, an accuracy of the touchscreen of the portable device, a connectivity of the portable device to a bridge device, and a connectivity of the portable device to a remote system.

In one method, the test comprises requesting an input from a user, and receiving the input from the user, in which case, the feature(s) (e.g., one of a sensitivity of a touchscreen of the portable device, an accuracy of the touchscreen of the portable device, a connectivity of the portable device to a bridge device, and a connectivity of the portable device to a remote system) is determined to be fulfilled by the portable device based on the input received from the user.

The method further comprises authorizing the portable device to communicate with the implantable medical device only if the feature(s) is fulfilled by the portable device, and denying authorization of the portable device to communicate with the implantable medical device if the feature(s) is not fulfilled by the portable device. If a plurality of features is to be tested on the portable device, the portable device may be authorized to communicate with the implantable medical device only if all of the features are fulfilled by the portable device.

The method may further comprise providing a graphical user interface that allows the portable device to communicate with the implantable medical device only if the portable device is authorized to communicate with the implantable medical device.

An optional method comprises detecting a modification in the portable device, wherein the modification is at least one of an installation of software or firmware, an upgrade of software or firmware, an enablement of a capability, and a disablement of a capability, and automatically de-authorizing the modified portable device from communicating with the implantable medical device when the modification is detected. In this case, the method may further comprise performing the test that determines whether the device configuration parameter(s) respectively corresponding to the feature(s) to be tested is fulfilled by the modified portable device, and authorizing the modified portable device to communicate with the implantable medical device if the feature(s) is fulfilled by the modified portable device.

Another optional method comprises transmitting, to a server, a request to download a configuration file, and receiving the configuration file from the server into the portable device. The configuration file includes the device configuration parameter(s) corresponding to the feature(s) to be tested on the portable device. In this case, the method may further comprise receive an updated configuration file from the server, which includes at least one updated device configuration parameter corresponding to at least one updated feature to be tested on the portable device, and de-authorizing the portable device when the updated configuration file is received. In this case, the method may further comprise performing a test that determines whether the updated device configuration parameter(s) respectively corresponding to the updated feature(s) to be tested is fulfilled by the portable device, and authorizing the portable device to communicate with the implantable medical device if the updated feature(s) is fulfilled by the portable device.

In accordance with a second aspect of the present inventions, a server for facilitating authorization of a portable device (e.g., a personal smart device) to communicate with an implantable medical device (e.g., an implantable pulse generator) is provided. The server comprises a memory storing a configuration checking program and a configuration file having at least one device configuration parameter respectively corresponding to at least one feature to be tested on the portable device, the configuration checking program, when executed by the portable device, performs a test that determines whether at least one device configuration parameter respectively corresponding to at least one feature to be tested on the portable device is fulfilled by the portable device, authorizes the portable device to communicate with the implantable medical device only if the feature(s) is fulfilled by the portable device, and denies authorization of the portable device to communicate with the implantable medical device if the feature(s) is not fulfilled by the portable device. The server further comprises a modem configured for remotely communicating with the portable device, and a controller configured for receiving a request from the portable device to download the configuration file and the configuration checking program, and for transmitting the configuration file and the configuration checking program to the portable device in response to the request.

The configuration file may include the device configuration parameter(s) respectively corresponding to the feature(s) to be tested on the portable device. The feature to be tested on the portable device may, e.g., be any of the previously listed features, and may be tested in the same manner described above. In an optional embodiment, the memory further stores the updated configuration file described above, in which case, the controller will be further configured for receiving a request from the portable device to download the updated configuration file, and for transmitting the updated configuration file to the portable device in response to the request.

In accordance with an aspect of the present disclosure a portable device (e.g., a personal smart device) in communication with a server is provided. The portable device comprises memory configured for storing a configuration checking program and a configuration file having at least one device configuration parameter respectively corresponding to at least one feature to be tested on the portable device, wherein the configuration checking program, when executed, performs a test that determines whether at least one device configuration parameter respectively corresponding to at least one feature to be tested on the portable device is fulfilled by the portable device, authorizes the portable device to communicate with an implantable medical device only if the feature(s) is fulfilled by the portable device, and denies authorization of the device to communicate with the implantable medical device if the feature(s) is not fulfilled by the portable device. The portable device further comprises a modem configured for remotely communicating with the server, and a controller configured for sending a request to the server to download the configuration file, receiving the configuration file in response to the request, and for executing the configuration checking program.

The configuration file may include the device configuration parameter(s) respectively corresponding to the feature(s) to be tested on the portable device. The feature to be tested on the portable device may, e.g., be any of the previously listed features, and may be tested in the same manner described above. In an optional embodiment, the memory is further configured for storing the updated configuration file described above, in which case, the controller will be further configured for sending a request from the portable device to download the updated configuration file, and for receiving the updated configuration file in response to the request.

Other and further aspects and features of the invention will be evident from reading the following detailed description of the preferred embodiments, which are intended to illustrate, not limit, the invention.

### BRIEF DESCRIPTION OF THE DRAWINGS

The drawings illustrate the design and utility of preferred embodiments of the present invention, in which similar elements are referred to by common reference numerals. In order to better appreciate how the above-recited and other advantages and objects of the present inventions are obtained, a more particular description of the present invention briefly described above will be rendered by reference to specific embodiments thereof, which are illustrated in the accompanying drawings. Understanding that these drawings depict only typical embodiments of the invention and are not therefore to be considered limiting of its scope, the invention will be described and explained with additional specificity and detail through the use of the accompanying drawings in which:
**Fig. 1** is a plan view of a tissue stimulation system for use in conjunction with the methods and systems of the present invention;
**Fig. 2** is an alternative embodiment of a remote tissue stimulation system for use in conjunction with the methods and systems of the present invention;
**Fig. 3** is a block diagram of system for authorizing a portable device to communicate with an implantable medical device of the tissue stimulation systems of **Figs. 2** and **3**;
**Fig. 4** is a block diagram of the internal components of the server shown in **Fig. 3**;
**Fig. 5** is a block diagram of the internal components of the portable device shown in **Fig. 3**;
**Fig. 6** illustrates an exemplary configuration file for authorizing the portable device shown in **Fig. 3**;
**Fig. 7** illustrates a list of features included in the configuration file shown in **Fig. 6**;
**Fig. 8** is a plan view of an exemplary graphical user interface (GUI) displayed on the portable device for controlling the implantable medical device shown in **Fig. 2** or **Fig. 3****;** and
**Fig. 9** is a flow diagram of a method for authorizing the portable device to communicate with the implantable medical device shown in **Fig. 2** or **Fig. 3****.**

### DETAILED DESCRIPTION OF THE EMBODIMENTS

The following description is provided in the context of a tissue stimulation system, e.g., a spinal cord stimulation system, a deep brain stimulation system, etc. However, it is to be understood that, while the methods and systems described herein lend themselves to tissue stimulation applications, the methods and systems, in their broadest aspects, are not so limited. Rather the methods and systems may be used with any type of portable device that communicates with an implantable medical device. For example, the methods and systems described herein may be used in conjunction with a pacemaker, an implantable defibrillator, a cochlear stimulator, a retinal stimulator, a stimulator configured to produce coordinated limb movement, a cortical stimulator, a peripheral nerve stimulator, a microstimulator, any other neural stimulator configured to treat urinary incontinence, sleep apnea, shoulder subluxation, headache, etc.

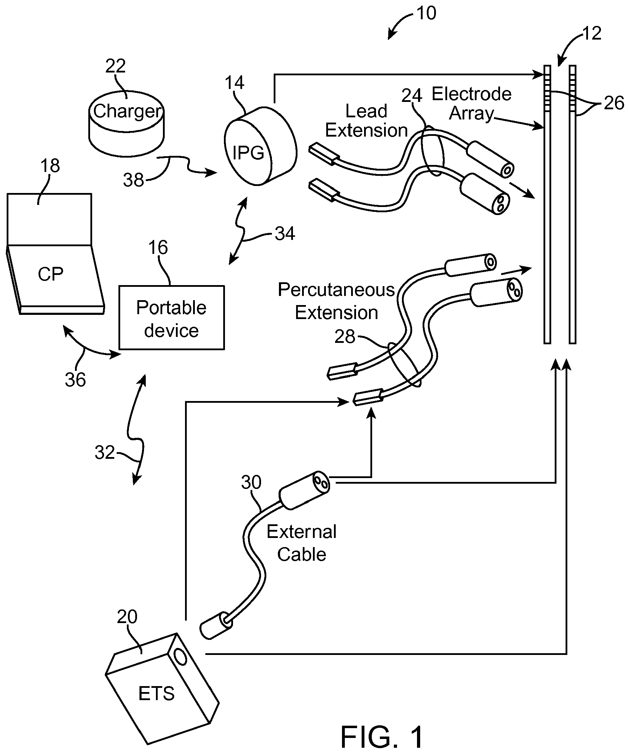

Turning to **Fig. 1****,** an exemplary tissue stimulation system 10 includes one or more (in this case, two) implantable stimulation leads 12, an implantable pulse generator (IPG) 14, a portable device 16, a clinician's programmer (CP) 18, an External Trial Stimulator (ETS) 20, and an external charger 22.

The portable device 16 is a personal smart device (PSD) 16 that the patient typically carries or uses for day-to-day activities, such as calling a friend, surfing the internet, sending or receiving messages, listening to music, etc. In the illustrated embodiment, the PSD 16 is hand-held in that it is small enough to be held in the palm of a hand. For example, the PSD 16 can be a smart phone, personal digital assistant (PDA), etc. In an alternative embodiment, the PSD 16 may be larger than a hand-held device, yet still easily portable. For example, in this case, the PSD 16 may be a tablet, iPad, etc.

Significant to the present inventions, the PSD 16 is configured for communicating with the IPG 14 upon authorization and installation of the programming software necessary for the PSD 16 to communicate with and program the IPG 14. The authorization includes checking whether the PSD 16 has sufficient features required to effectively and safely control the IPG 14, as discussed below in greater detail. Once the PSD 16 is authorized, the PSD 16 can control the IPG 14 by adjusting stimulation parameters, turning on and off stimulation, checking battery status, etc.

The IPG 14 is physically connected via one or more percutaneous lead extensions 24 to the stimulation leads 12, which carry a plurality of electrodes 26 arranged in an array. In the illustrated embodiment, the stimulation leads 12 are percutaneous leads, and to this end, the electrodes 26 are arranged in-line along the stimulation leads 12. In alternative embodiments, the electrodes 26 may be arranged in a two-dimensional pattern on a single paddle lead. The IPG 14 includes pulse generation circuitry that delivers electrical stimulation energy in the form of a pulsed electrical waveform (i.e., a temporal series of electrical pulses) to the electrode array 26 in accordance with a set of stimulation parameters.

The ETS 20 may also be physically connected via percutaneous lead extensions 28 and external cable 30 to the stimulation leads 12. The ETS 20, which has similar pulse generation circuitry as that of the IPG 14, also delivers electrical stimulation energy in the form of a pulsed electrical waveform to the electrode array 26 in accordance with a set of stimulation parameters. The major difference between the ETS 20 and the IPG 14 is that the ETS 20 is a non-implantable device that is used on a trial basis, after the stimulation leads 12 have been implanted and prior to implantation of the IPG 14, to test the responsiveness of the stimulation that is to be provided. Thus, any functions described herein with respect to the IPG 14 can likewise be performed with respect to the ETS 20.

The PSD 16 may be used to telemetrically control the ETS 20 via a bi-directional RF communications link 32. The IPG 14 and the stimulation leads 12 are configured to be implanted within a patient, such that the electrodes 26 are in contact with the tissue, e.g., spinal cord tissue or brain tissue, to be stimulated. Once the IPG 14 and stimulation leads 12 are implanted, the PSD 16 may be used to telemetrically control the IPG 14 via a bi-directional RF communications link 34. Such control allows the IPG 14 to be turned on or off, and to be programmed with different stimulation parameter sets. The PSD 16 may also be operated to modify the programmed stimulation parameters to actively control the characteristics of the electrical stimulation energy output by the IPG 14.

The CP 18 provides clinician detailed stimulation parameters for programming the IPG 14 and ETS 20 in the operating room and in follow-up sessions. The CP 18 may perform this function by indirectly communicating with the IPG 14 or ETS 20, through the PSD 16, via an IR communications link 36. Alternatively, the CP 18 may directly communicate with the IPG 14 or ETS 20 via an RF communications link (not shown). The clinician detailed stimulation parameters provided by the CP 18 are also used to program the PSD 16, so that the stimulation parameters can be subsequently modified by operation of the PSD 16 in a stand-alone mode (i.e., without the assistance of the CP 18).

The external charger 22 is a portable device used to transcutaneously charge the IPG 14 via an inductive link 38. Once the IPG 14 has been programmed, and its power source has been charged by the external charger 22 or otherwise replenished, the IPG 14 may function as programmed without the device 16 or CP 18 being present. For purposes of brevity, further details of the IPG 14, CP 18, ETS 20, and external charger 22 will not be described herein. Details of exemplary embodiments of these devices are disclosed in U.S. Patent No. 6,895,280.

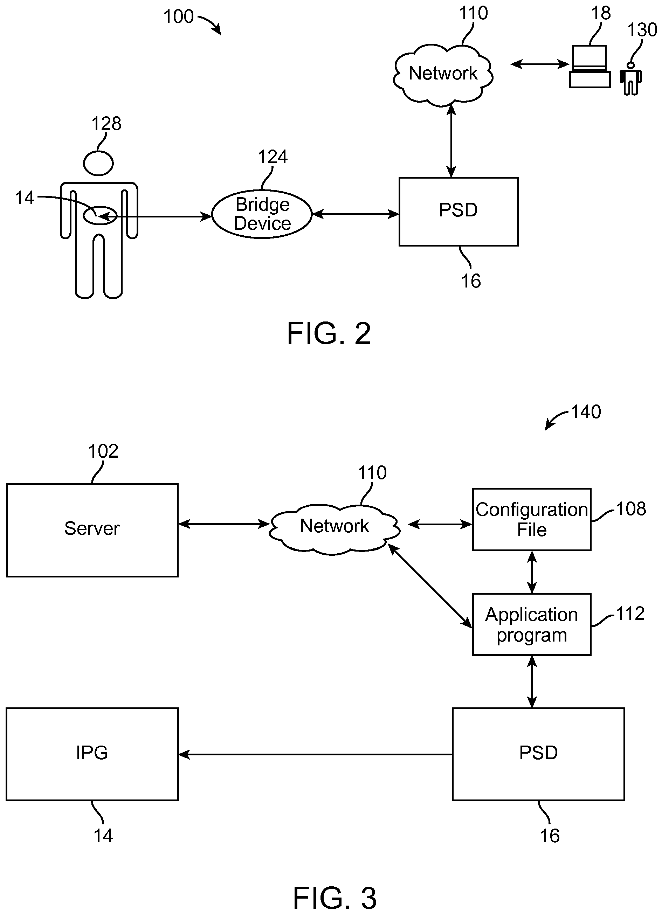

Turning to **Fig. 2**, a remote tissue stimulation system 100 will be described herein. The system 100 is similar to the system 10 described above, with the exception that the PSD 16 may indirectly communicate with the IPG 14 (or alternatively the ETS 20) via a bridge device 124, and the CP 18 (operated by a clinician 130) may remotely communicate with the PSD 16 via a network 110.

In the illustrated embodiment, the bridge device 124 is a telemetry wand that can be conveniently placed locally with respect to a patient 128 (e.g., within four feet) to enhance or supplement communication between the PSD 16 and the IPG 14. The network 110 that allows communication between the PSD 16 and the network 110 can be a local area network (LAN), wide area network (WAN), world wide web (www), public switched telephone network (PSTN), cellular telephone network, or any other terrestrial or satellite network appropriate for use with the CP 18. These devices (i.e., CP 18 and PSD 16) can communicate with each other using any known, related art and/or later developed protocols.

Referring to **Fig. 3**, a system 140 for facilitating authorization of the PSD 16 to communicate with the IPG 16 is described. As mentioned above, the authorization includes checking whether the PSD 16 can act as a control device for the IPG 14. In general, the system 140 includes a server 102 that communicates with the PSD 16 via the network 110. For example, communication between the PSD 16 and the server 102 may be over phone lines, over the web, etc.

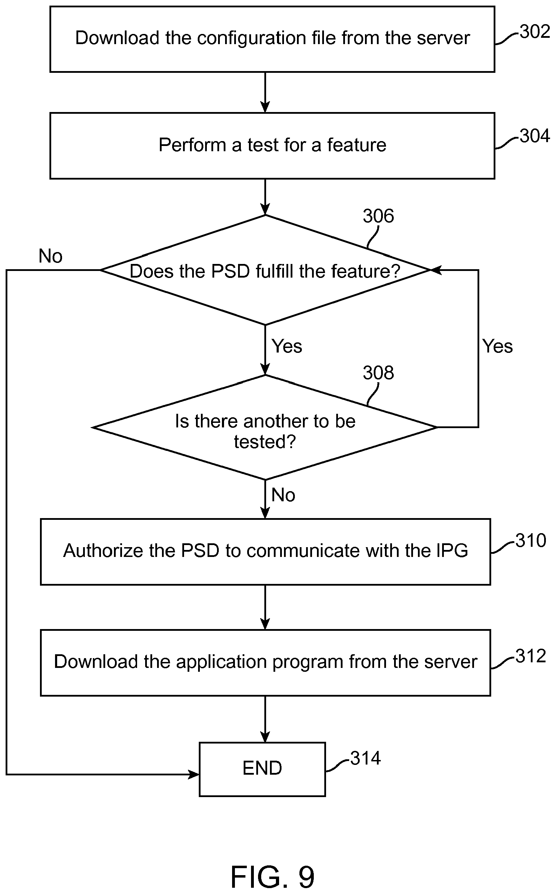

The PSD 16 is configured for communicating with the server 102 via the network 110 for retrieving a configuration file 108 and a configuration checking program that uses the configuration file 108 to perform the test. In a preferred embodiment, the configuration checking program is an application program 112 which when executed by the PSD 16, checks whether the PSD 16 has sufficient features to communicate with and further control the IPG 14. If the PSD 16 does have such sufficient features, the application program 112 authorizes the PSD 16 to communicate with the IPG 14. In such cases, the application program 112 provides a notification to the patient 128 indicating that the PSD 16 is authorized to communicate with the implantable IPG 14. Else, the application program 112 denies the authorization of the PSD 16 to communicate with the IPG 14. In instances where the PSD 16 is authorized, the application program 112 provides a graphical user interface to the patient 128 for controlling the IPG 14 and will be discussed below in conjunction with **Fig. 9**. In an alternative case where a configuration checking program separate from the application program 112 is utilized, the PSD 16 may be further configured for retrieving or downloading the application program 112 from the server 102 only after the PSD 16 is authorized to communicate and control the IPG 14.

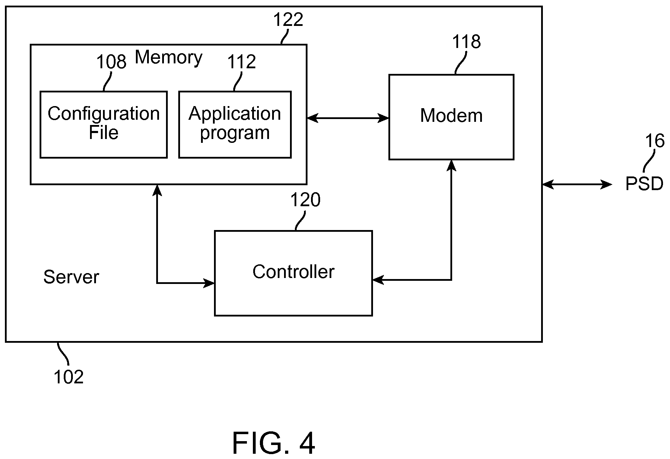

Referring to **Fig. 4**, the internal components of the server 102 will now be described. The server 102 generally includes memory 122 configured for storing the configuration file 108 and the application program 112, a modem 118 configured for remotely communicating with the PSD 16, and a controller 120 configured for receiving a request from the PSD 16 to download the configuration file 108 and the application program 112, and for transmitting the configuration file 108 and the application program 112 to the PSD 16 in response to the request. As mentioned above, the application program 112 performs a test to determine whether the PSD 16 is capable of communicating with the IPG 14, and based on the test performed, authorizes the PSD 16 to communicate with the IPG 14.

The memory 122 may be any volatile, non-volatile, fixed, removable, magnetic, optical, or electrical media, such as a RAM, ROM, CD-ROM, hard disk, removable magnetic disk, memory cards or sticks, NVRAM, EEPROM, flash memory, and the like. The controller 120 may include a microprocessor, a controller, a digital signal processor (DSP), an application specific integrated circuit (ASIC), a field programmable gate array (FPGA), or other discrete or integrated logic circuitry.

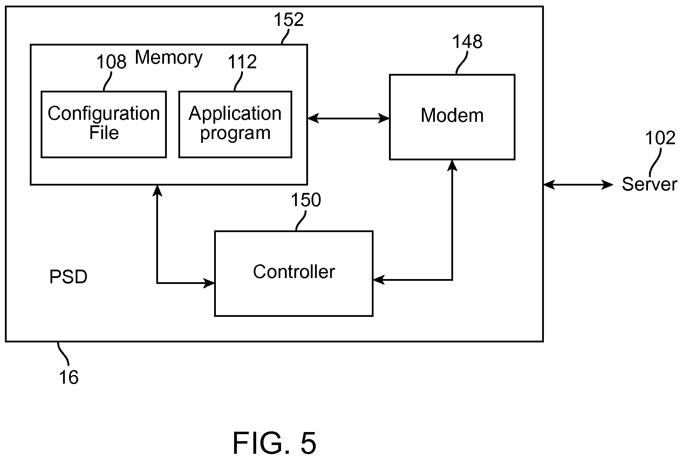

Referring to **Fig. 5**, the internal components of the PSD 16 will now be described. The PSD 16 generally includes memory 152 configured for storing the configuration file 108 and the application program 112, a modem 148 configured for remotely communicating with the server 102, and a controller 150 configured for sending a request to the server 102 to download the configuration file 108, for receiving the configuration file 108 in response to the request, and for executing the application program 112 (discussed below in greater detail).

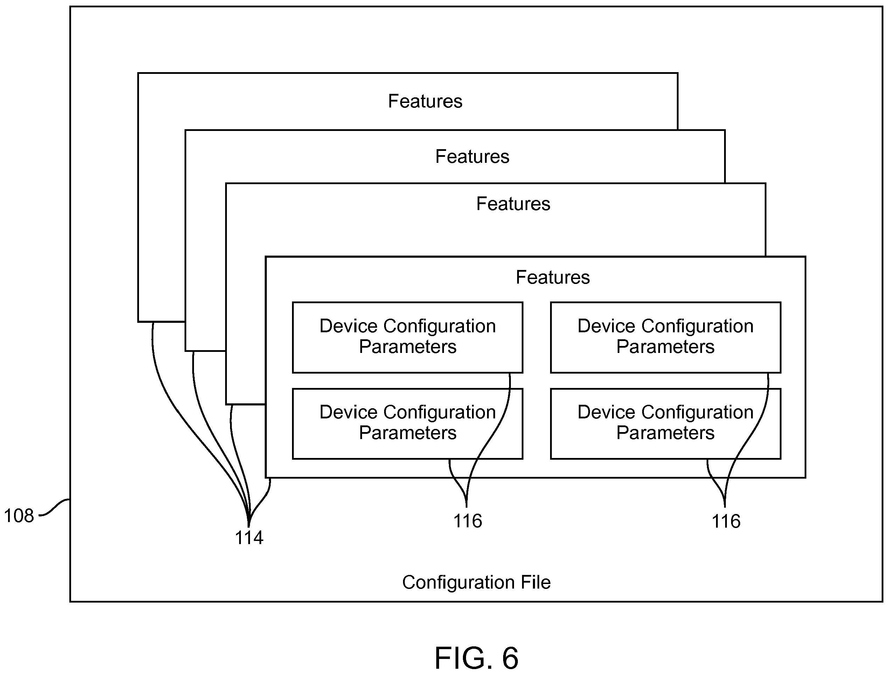

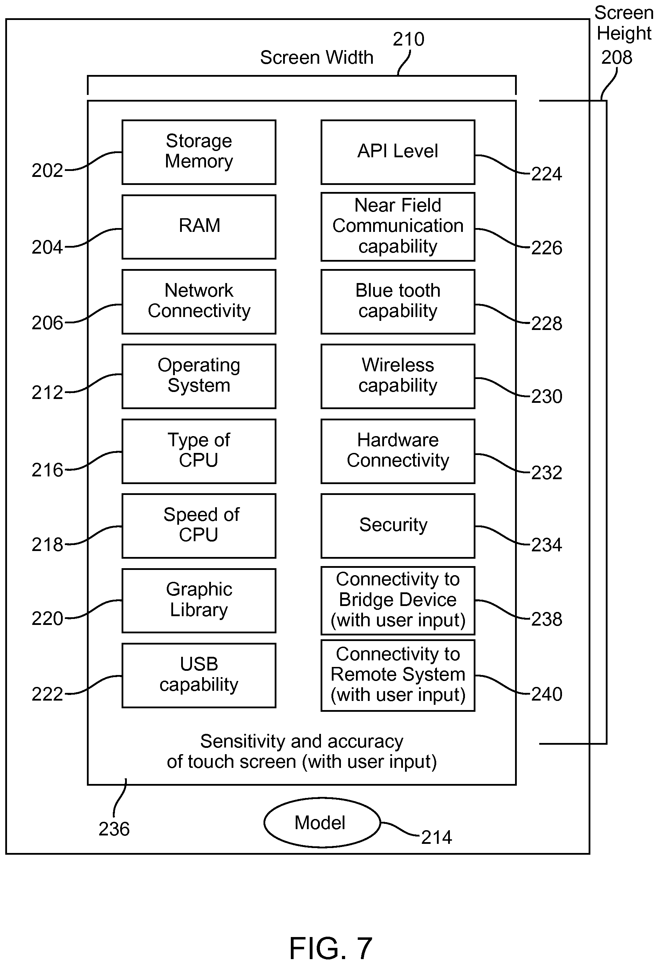

As shown in **Fig. 6**, the configuration file 108 is an input file that includes one or more features 114 to be tested on the PSD 16. Few such examples of the features 114 (shown in **Fig. 7**) include a storage memory 202 of the PSD 16, a random access memory 204 of the PSD 16, a capability 206 of the PSD 16 to connect to a type of a network, a screen height 208 of the PSD 16, a screen width 210 of the PSD 16, an operating system 212 of the PSD 16, a model 214 of the PSD 16, a type of central processing unit (CPU)216 of the PSD 16, a speed of central processing unit 218 of the PSD 16, a type of graphic library 220 of the PSD 16, a Universal Serial Bus (USB) capability 222 of the PSD 16, an Application Program Interface (API) level 224 of the PSD 16, a near field communication (NFC) capability 226 of the PSD 16, a Bluetooth® capability 228 of the PSD 16, a wireless capability 230 of the PSD 16, a hardware connectivity performance 232 of the PSD 16, a type of security capability 234 of the PSD 16, a sensitivity and accuracy of a touchscreen 236 of the PSD 16, a connectivity 238 of the PSD 16 to the bridge device 124, and a connectivity 240 of the PSD 16 to a remote system, such as the remote CP 18 in **Fig. 2****.**

As briefly discussed above, the configuration file 108 is input to the application program 112 and the application program 112 performs the test. For some of the features 114 listed above, the application program 112 requires an input from the patient 128 to determine whether that particular feature is fulfilled by the PSD 16 or not. Such features may be the sensitivity and accuracy 236 of the touchscreen of the PSD 16, the connectivity 238 of the PSD 16 to the bridge device 124, and the connectivity 240 of the PSD 16 to the remote system. To this end, the application program 112 requests an input from the patient 128 and then receives the input from the patient 128. For example, for the sensitivity and accuracy of the touch screen feature 236, the application program 112 requests the patient 128 to touch one or more areas in the screen of the PSD 16. For the connectivity 238 to the bridge device 124 feature, the application program 112 requests the patient 128 to turn on the bridge device 124, so that the test can be performed to verify the communication link between the PSD 16 and the bridge device 124. For the connectivity 240 to the remote system 240 feature, the application program 112 verifies the round trip communication link from the remote CP 18 to the PSD 16, the bridge device 124, the IPG 14 and back again to the CP 18. This test may require acknowledgement from the patient 128 to accept connection from the remote system or turning on the bridge device 124.

Each of the features 114 is associated with one or more configuration parameters 116. In some cases, the configuration parameters 116 may define minimum/maximum values or versions of the features 114 to be fulfilled by the PSD 16. In other cases, the configuration parameters 116 may be defined to check the availability of the features 114 or to determine capability of the available features 114. The configuration parameters 116 specified in the configuration file 108 for a particular feature 114, are matched against the actual parameters of the PSD 16 and based on the matching, the application program 112 determines whether that particular feature 114 is fulfilled by the PSD 16. The configuration parameters 116 for each of the features 114 and the test to be performed for these features 114 will now be discussed in greater detail.

For the storage memory feature 202, the configuration parameters may be minimum storage of the PSD 16 and available storage memory. Accordingly, the application program 112 checks whether the PSD 16 has sufficient internal memory to store the application program 112 or any associated applications required for performing the test or for controlling the IPG 14. For the RAM feature 204, the configuration parameters may be minimum RAM and available RAM. Accordingly, the application program 112 checks whether the PSD 16 has sufficient RAM required by the CPU to execute tasks, such as executing the application program 112. For the network connectivity feature 206, the configuration parameters may be whether the PSD 16 is capable of connecting to one or more networks, such as Wi-Fi, 2.75G, 3G, 4G, or the like. Accordingly, the application program 112 determines whether the PSD 16 is capable of connecting to the networks for communicating with devices, such as the remote CP 18. The network capability of the PSD 16 allows the PSD 16 to receive programming commands/data remotely from the devices, such as the remote CP 18. For the screen height and width features 208, 210 respectively, the configuration parameters may be minimum screen height and width. Accordingly, the application program 112 checks whether the PSD 16 has sufficient screen height 204 and screen width 202 to display data.

For the operating system feature 212, the configuration parameters may be the type of operating system. Accordingly, the application program 112 checks which operating system (such as Android™, windows®, etc.) is running on the PSD 16 and whether that operating system is desired for executing the application program 112. For the model feature 214, the configuration parameters may be model number/ID of the PSD 16. Accordingly, the application program 112 checks the model of the PSD 16 and further checks whether that model is compatible with the application program 112. For the type of CPU feature 216, the configuration parameters may be type of processor. Accordingly, the application program 112 checks the type of the processor and whether that processor is capable of processing or executing the tasks as required. For example, application program 112 checks whether the processor is a single core, dual core, quad core, ARM, x86, MIPS, etc. For the speed of the CPU feature 218, the configuration parameter may be minimum speed of the processor. Accordingly, the application program 112 determines whether the speed of the CPU of the PSD 16 is sufficient for running the above mentioned applications on the PSD 16. For the graphic library feature 220, the configuration parameter may be which graphic library is supported by the PSD 16. Accordingly, the application program 112 further checks whether the PSD 16 has desired graphic library to render graphics to a display.

For the USB capability feature 222, the configuration parameter may be whether the PSD 16 has USB port or not. Accordingly, the application program 112 checks whether the USB is capable of communicating with other devices or is it only for charging purposes.

For the API level feature 224, the configuration parameter may be minimum version of operating system running on the PSD 16. Accordingly, the application program 112 checks which version of operating system is running on the PSD 16 and further ascertain what is the minimum version required to run various applications, such as the application program 112. The application program 112 also determines whether any software application is compatible with the PSD 16 prior to installing it.

For the NFC capability feature 226, the configuration parameter may be whether the PSD 16 has an NFC controller. Accordingly, the application program 112 determines whether the PSD 16 is capable of communicating with other devices via the NFC. For the Bluetooth® capability feature 228, the configuration parameter may be whether the PSD 16 has Bluetooth®. Accordingly, the application program 112 checks whether the PSD 16 can share the data via Bluetooth®. For the wireless capability feature 230, the configuration parameter may be whether the PSD 16 is capable of communicating through wireless links, such as Bluetooth®, Wi-Fi, or WLAN or other wireless networks. Accordingly, the application program 112 checks whether the PSD 16 has sufficient means/ways through which the PSD 16 can communicate with devices through wireless links. For the hardware connectivity performance feature 232, the configuration parameter may be processing power of the PSD 16. Accordingly, the application program 112 checks how instantly the test can be performed and defines a range of communication and the like. For the security feature 234, the configuration parameter may be type of security features such as PIN, password, pattern or the like. Accordingly, the application program 112 checks whether the PSD 16 has sufficient ways to secure the PSD 16 and the application program 112, from being misused.

For the sensitivity and accuracy of the touch screen feature 236, the configuration parameter may be response time, and accuracy. Accordingly, the application program 112 checks responsiveness of the touch screen when a portion of the screen is touched and also checks whether the portion touched by the patient 128 has been correctly interpreted by the CPU or not. For the connectivity feature 238 to the bridge device 124, the configuration parameter may be whether the PSD 16 can be connected to the bridge device 124. Accordingly, the application program 112 checks whether the PSD 16 has sufficient ways of connecting the PSD 16 to the bridge device 124. For the connectivity feature 240 to the remote system, the configuration parameter may be whether the PSD 16 can be connected to the remote system such as server 102 or CP 18. Accordingly, application program 112 checks whether the PSD 16 has sufficient means of connecting the PSD 16 to the remote system.

For a person skilled in the art, it is understood that the configuration parameters and corresponding tests as mentioned above are exemplary in nature and there can be many more types of configuration parameters and tests for each of the features 114. The configuration file 108 may store these features 114 and corresponding configuration parameters 116 in any desired format.

In some instances, the application program 112 may be configured to check a minimum number of features 114 (such as 2) to be fulfilled by the PSD 16, while in other instances, the application program 112 may be configured to check whether all the features as discussed above are fulfilled by the PSD 16 or not. For a person skilled in the art, it is understood that the application program 112 may be configured in any manner as desired.

In an optional embodiment, the application program 112 may monitor the PSD 16 on regular intervals for any modifications. For example, the modification may be in the terms of an installation of software or firmware, an upgrade of software or firmware, an enablement of a capability, or a disablement of a capability. The application program 112 automatically de-authorizes the PSD 16 from communicating with the IPG 14 if the application program 112 determines that the PSD 16 is modified in any way. Post de-authorization, the application program 112 re-performs the test to determine whether the features 114 are fulfilled by the modified PSD. If the features are fulfilled by the modified PSD, then the modified PSD is authorized to communicate with the IPG 14. Otherwise, the modified PSD 16 is not authorized to communicate with the IPG 14. The application program 112 may also monitor the PSD 16 when the user deactivates or removes a feature required by the configuration file 108. For example, if the configuration file 108 requires GPS capability, an accelerometer, Wi-Fi, NFC, or a SIM card, and the user disables or otherwise removes one of these features, the application program 112 may automatically de-authorize the PSD 16 until the feature(s) is enabled or otherwise re-installed on the PSD 16.

In another optional embodiment, the PSD 16 receives an updated configuration file and then stores the updated configuration file in the memory 152. The updated configuration file includes one or more updated device configuration parameters corresponding to one or more updated features to be tested on the PSD 16. The updated configuration file is used by the application program 112 to automatically de-authorize the PSD 16 to communicate with the IPG 14. Similar to the above, the test is performed again to determine whether the updated feature(s) are fulfilled by the PSD 16. Based on the test performed, the PSD 16 is authorized to communicate with the IPG 14 only when the PSD 16 fulfills the feature.

In other embodiments, the configuration checking program can be any program (i.e., separate than the application program 112), which when executed by the PSD 16, performs the test and authorizes the PSD 16 using the configuration file 108 as mentioned above. In such cases, the application program 112 can be downloaded from the server 102 after the test is performed. The application program 112 is received only after the PSD 16 is authorized to communicate with IPG 14. The application program 112 upon execution provides the graphical user interface to communicate with the IPG 14.

Turning to **Fig. 9**, a method of using the system 140 to authorize the PSD 16 to communicate with the IPG 14 is described. To begin with, the configuration file 108 and the application program 112 are downloaded from the server 102 to the PSD 16 (step 302). Once the configuration file 108 and the application program 112 are appropriately downloaded into the PSD 16, the configuration file 108 is input to the application program 112 and the application program 112 performs a test for one of the features of the PSD 16 (step 304). Then, the application program 112 checks whether the feature is fulfilled by the PSD 16 (step 306). During the test, the application program 112 matches the feature requirement as specified in the configuration file 108 with the actual feature of the PSD 16. Based on the matching, if it is determined that the feature of the PSD 16 is not fulfilled, the method ends (step 314). In such cases, the PSD 16 is not authorized to communicate with the IPG 14. But if the feature is fulfilled by the PSD 16, then the application program 112 checks whether there is another feature to be tested for the PSD 16 (step 308). If so, the method returns to step 306 to check whether the other feature is fulfilled by the PSD 16. This process is continued until all features of the PSD 16 are tested. In case there is no further feature to be tested, then the application program 112 authorizes the PSD 16 to communicate with the IPG 14 (step 310).

Once the PSD 16 is authorized to communicate with the IPG 14, the application program 112 provides a graphical user interface (GUI) that allows the PSD 16 to communicate with the IPG 14 (step 312). The GUI allows the patient 128 to adjust one or more stimulation parameters as required. For example, the GUI allows the user to modify the stimulation parameters of the stimulation energy output by the IPG 14 to the electrodes 26 by adjusting at least one of an electrode combination, pulse amplitude, pulse width, or pulse rate. The method then ends (step 314).

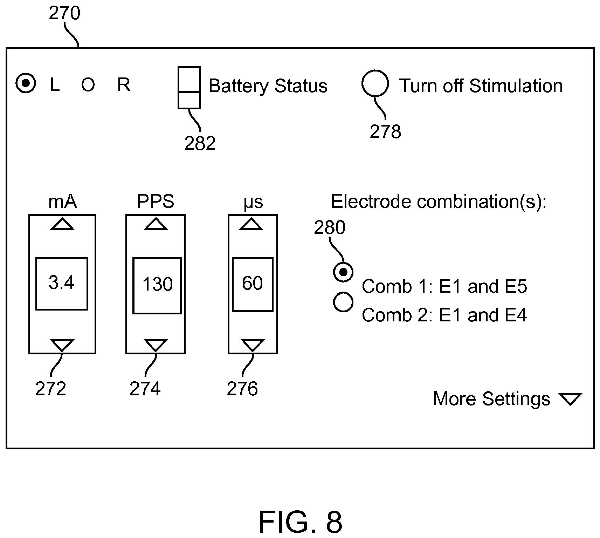

One such exemplary GUI 270 that includes pulse amplitude control 272, pulse rate control 274 and pulse width control 276, and one or more icons 278, and 280 is shown in **Fig. 8**. These controls 272, 274, and 276 can be actuated by the patient 128 to increment or decrement any of the electrical pulse parameters - pulse amplitude, pulse rate, pulse width respectively using up and down arrows. In the similar manner, the icon 280 can be actuated to select the desired electrode combination. The icon 278 can be actuated to turn the IPG 14 on or off. Moreover, the GUI 270 also includes an icon 282 indicating battery status of the IPG 14.

## Claims

1. A method for authorizing a portable device to communicate with an implantable medical device, the method comprising:
performing a test that determines whether at least one device configuration parameter respectively corresponding to at least one feature to be tested on the portable device is fulfilled by the portable device, wherein the at least one feature is one or more features selected from the group consisting of:
a storage memory of the portable device, a random access memory of the portable device, a screen height of the portable device, a screen width of the portable device, a model of the portable device, a speed of central processing unit of the portable device, a USB capability of the portable device, a near field communication capability of the portable device, a Bluetooth® capability of the portable device, a sensitivity of a touchscreen of the portable device, and an accuracy of the touchscreen of the portable device;
authorizing the portable device to communicate with the implantable medical device only if the at least one feature is fulfilled by the portable device; and
denying authorization of the portable device to communicate with the implantable medical device if the at least one feature is not fulfilled by the portable device.

2. The method of claim 1, further comprising providing a graphical user interface that allows the portable device to communicate with the implantable medical device only if the portable device is authorized to communicate with the implantable medical device.

3. The method of claim 1, wherein the portable device comprises a personal smart device.

4. The method of claim 1, wherein performing the test comprises requesting an input from a user, and receiving the input from the user, wherein the at least one feature is determined to be fulfilled by the portable device based on the input received from the user.

5. The method of claim 4, wherein the feature to be tested is one of a sensitivity of a touchscreen of the portable device, an accuracy of the touchscreen of the portable device, a connectivity of the portable device to a bridge device, and a connectivity of the portable device to a remote system.

6. The method of claim 1, further comprising:
detecting a modification in the portable device, wherein the modification is at least one of an installation of software or firmware, an upgrade of software or firmware, an enablement of a capability, and a disablement of a capability; and
automatically de-authorizing the modified portable device from communicating with the implantable medical device when the modification is detected.

7. The method of claim 6, further comprising:
performing the test that determines whether the at least one device configuration parameter respectively corresponding to the at least one feature to be tested is fulfilled by the modified portable device; and
authorizing the modified portable device to communicate with the implantable medical device if the at least one feature is fulfilled by the modified portable device.

8. The method of claim 1, further comprising:
transmitting, to a server, a request to download a configuration file; and
receiving the configuration file from the server into the portable device, wherein the configuration file includes the at least one device configuration parameter corresponding to the at least one feature to be tested on the portable device.

9. The method of claim 8, further comprising:
receiving an updated configuration file from the server, wherein the updated configuration file includes at least one updated device configuration parameter corresponding to at least one updated feature to be tested on the portable device; and
de-authorizing the portable device when the updated configuration file is received.

10. The method of claim 9, further comprising:
performing a test that determines whether the at least one device configuration parameter respectively corresponding to the at least one updated feature to be tested is fulfilled by the portable device; and
authorizing the portable device to communicate with the implantable medical device if the at least one updated feature is fulfilled by the portable device.

11. The method of claim 1, wherein the at least one device configuration parameter comprises a plurality of device configuration parameters, the at least one feature comprises a plurality of features, and the portable device is authorized to
communicate with the implantable medical device only if allof the features are fulfilled by the portable device.

12. A server for facilitating authorization of a portable device to communicate with an implantable medical device, wherein the server comprises:
a memory storing a configuration checking program and a configuration file having at least one device configuration parameter respectively corresponding to at least one feature to be tested on the portable device, wherein the configuration checking program, when executed by the portable device, performs a test that determines whether the at least one device configuration parameter is fulfilled by the portable device, authorizes the portable device to communicate with the implantable medical device only if the at least one feature is fulfilled by the portable device, and denies authorization of the portable device to communicate with the implantable medical device if the at least one feature is not fulfilled by the portable device;
a modem configured for remotely communicating with the portable device; and
a controller configured for receiving a request from the portable device to download the configuration file and the configuration checking program, and for transmitting the configuration file and the configuration checking program to the portable device in response to the request.

13. The server of claim 12, wherein the feature to be tested on the portable device is one of a storage memory of the portable device, a random access memory of the portable device, a capability of the portable device to connect to a type of a network, a screen height of the portable device, a screen width of the portable device, an operating system of the portable device, a model of the portable device, a type of central processing unit of the portable device, a speed of central processing
unit of the portable device, a type of graphic library of the portable device, a USB capability of the portable device, an API level of the portable device, a near field communication capability of the portable device, a Bluetooth® capability of the portable device, a wireless capability of the portable device, a hardware connectivity performance of the portable device, a type of security capability of the portable device, a sensitivity of a touchscreen of the portable device, an accuracy of the touchscreen of the portable device, a connectivity of the portable device to a bridge device, and a connectivity of the portable device to a remote system.

14. The server of claim 12, wherein the feature to be tested is one of a sensitivity of a touchscreen of the portable device, an accuracy of the touchscreen of the portable device, a connectivity of the portable device to a bridge device, and a connectivity of the portable device to a remote system.

## Patentansprüche

1. Verfahren zum Autorisieren eines tragbaren Geräts, mit einem implantierbaren Medizinprodukt zu kommunizieren, wobei das Verfahren aufweist:
Durchführen eines Tests, der bestimmt, ob mindestens ein Gerätekonfigurationsparameter, der jeweils mindestens einem Merkmal entspricht, das auf dem tragbaren Gerät zu testen ist, durch das tragbare Gerät erfüllt wird, wobei das mindestens eine Merkmal ein oder mehrere Merkmale ist, die aus der Gruppe ausgewählt sind, die besteht aus:
einem Speicher des tragbaren Geräts, einem Arbeitsspeicher des tragbaren Geräts, einer Bildschirmhöhe des tragbaren Geräts, einer Bildschirmbreite des tragbaren Geräts, einem Modell des tragbaren Geräts, einer Geschwindigkeit des Prozessors des tragbaren Geräts, einer USB-Fähigkeit des tragbaren Geräts, einer Nahfeldkommunikationsfahigkeit des tragbaren Geräts, einer Bluetooth®-Fähigkeit des tragbaren Geräts, einer Empfindlichkeit eines Touchscreens des tragbaren Geräts und einer Genauigkeit des Touchscreens des tragbaren Geräts;
Autorisieren des tragbaren Geräts, mit dem implantierbaren Medizinprodukt nur zu kommunizieren, wenn das mindestens eine Merkmal durch das tragbare Gerät erfüllt wird; und
Verweigern der Autorisierung des tragbaren Geräts, mit dem implantierbaren Medizinprodukt zu kommunizieren, wenn das mindestens eine Merkmal nicht durch das tragbare Gerät erfüllt wird.

2. Verfahren nach Anspruch 1, das ferner aufweist: Bereitstellen einer grafischen Benutzerschnittstelle, die dem tragbaren Gerät ermöglicht, mit dem implantierbaren Medizinprodukt nur zu kommunizieren, wenn das tragbare Gerät autorisiert ist, mit dem implantierbaren Medizinprodukt zu kommunizieren.

3. Verfahren nach Anspruch 1, wobei das tragbare Gerät ein persönliches Smart Device aufweist.

4. Verfahren nach Anspruch 1, wobei das Durchführen des Tests aufweist: Anfordern einer Eingabe von einem Benutzer und Empfangen der Eingabe vom Benutzer, wobei das mindestens eine Merkmal auf der Grundlage der vom Benutzer empfangenen Eingabe als durch das tragbare Gerät erfüllt bestimmt wird.

5. Verfahren nach Anspruch 4, wobei das zu testende Merkmal eine Empfindlichkeit eines Touchscreens des tragbaren Geräts, eine Genauigkeit des Touchscreens des tragbaren Geräts, eine Konnektivität des tragbaren Geräts mit einer Brückenvorrichtung oder eine Konnektivität des tragbaren Geräts mit einem Fernsystem ist.

6. Verfahren nach Anspruch 1, das ferner aufweist:
Detektieren einer Modifizierung im tragbaren Gerät, wobei die Modifizierung eine Installation von Software oder Firmware, ein Upgrade von Software oder Firmware, eine Aktivierung einer Fähigkeit und/oder eine Deaktivierung einer Fähigkeit ist; und
automatisches Deautorisieren des modifizierten tragbaren Geräts, mit dem implantierbaren Medizinprodukt zu kommunizieren, wenn die Modifizierung detektiert wird.

7. Verfahren nach Anspruch 6, das ferner aufweist:
Durchführen des Tests, der bestimmt, ob der mindestens eine Gerätekonfigurationsparameter, der jeweils dem mindestens einen zu testenden Merkmal entspricht, durch das modifizierte tragbare Gerät erfüllt wird; und
Autorisieren des modifizierten tragbaren Geräts, mit dem implantierbaren Medizinprodukt zu kommunizieren, wenn das mindestens eine Merkmal durch das modifizierte tragbare Gerät erfüllt wird.

8. Verfahren nach Anspruch 1, das ferner aufweist:
zu einem Server erfolgendes Senden einer Anforderung, eine Konfigurationsdatei herunterzuladen; und
Empfangen der Konfigurationsdatei vom Server im tragbaren Gerät, wobei die Konfigurationsdatei den mindestens einen Gerätekonfigurationsparameter aufweist, der dem mindestens einen Merkmal entspricht, das auf dem tragbaren Gerät zu testen ist.

9. Verfahren nach Anspruch 8, das ferner aufweist:
Empfangen einer aktualisierten Konfigurationsdatei vom Server, wobei die aktualisierte Konfigurationsdatei mindestens einen aktualisierten Gerätekonfigurationsparameter aufweist, der mindestens einem aktualisierten Merkmal entspricht, das auf dem tragbaren Gerät zu testen ist; und
Deautorisieren des tragbaren Geräts, wenn die aktualisierte Konfigurationsdatei empfangen wird.

10. Verfahren nach Anspruch 9, das ferner aufweist:
Durchführen eines Tests, der bestimmt, ob der mindestens eine Gerätekonfigurationsparameter, der jeweils dem mindestens einen zu testenden aktualisierten Merkmal entspricht, durch das tragbare Gerät erfüllt wird; und
Autorisieren des tragbaren Geräts, mit dem implantierbaren Medizinprodukt zu kommunizieren, wenn das mindestens eine aktualisierte Merkmal durch das tragbare Gerät erfüllt wird.

11. Verfahren nach Anspruch 1, wobei der mindestens eine Gerätekonfigurationsparameter mehrere Gerätekonfigurationsparameter aufweist, das mindestens eine Merkmal mehrere Merkmale aufweist und das tragbare Gerät autorisiert wird, mit dem implantierbaren Medizinprodukt nur zu kommunizieren, wenn alle Merkmale durch das tragbare Gerät erfüllt werden.

12. Server zum Erleichtern der Autorisierung eines tragbaren Geräts, mit einem implantierbaren Medizinprodukt zu kommunizieren, wobei der Server aufweist:
einen Speicher, der ein Konfigurationsprüfprogramm und eine Konfigurationsdatei mit mindestens einem Gerätekonfigurationsparameter speichert, der jeweils mindestens einem Merkmal entspricht, das auf dem tragbaren Gerät zu testen ist, wobei das Konfigurationsprüfprogramm bei Ausführung durch das tragbare Gerät einen Test durchführt, der bestimmt, ob der mindestens eine Gerätekonfigurationsparameter durch das tragbare Gerät erfüllt wird, das tragbare Gerät autorisiert, mit dem implantierbaren Medizinprodukt nur zu kommunizieren, wenn das mindestens eine Merkmal durch das tragbare Gerät erfüllt wird, und Autorisierung des tragbaren Geräts verweigert, mit dem implantierbaren Medizinprodukt zu kommunizieren, wenn das mindestens eine Merkmal nicht durch das tragbare Gerät erfüllt wird; ein Modem, das zum Fernkommunizieren mit dem tragbaren Gerät konfiguriert ist; und
eine Steuerung, die zum Empfangen einer Anforderung vom tragbaren Gerät, die Konfigurationsdatei und das Konfigurationsprüfprogramm herunterzuladen, und
zum Senden der Konfigurationsdatei und des Konfigurationsprüfprogramms zum tragbaren Gerät als Reaktion auf die Anforderung konfiguriert ist.

13. Server nach Anspruch 12, wobei das auf dem tragbaren Gerät zu testende Merkmal ein Speicher des tragbaren Geräts, ein Arbeitsspeicher des tragbaren Geräts, eine Fähigkeit des tragbaren Geräts, sich mit einer Art eines Netzwerks zu verbinden, eine Bildschirmhöhe des tragbaren Geräts, eine Bildschirmbreite des tragbaren Geräts, ein Betriebssystem des tragbaren Geräts, ein Modell des tragbaren Geräts, eine Art von Prozessor des tragbaren Geräts, eine Geschwindigkeit des Prozessors des tragbaren Geräts, eine Art von grafischer Bibliothek des tragbaren Geräts, eine USB-Fähigkeit des tragbaren Geräts, eine API-Qualität des tragbaren Geräts, eine Nahfeldkommunikationsfähigkeit des tragbaren Geräts, eine Bluetooth®-Fähigkeit des tragbaren Geräts, eine Drahtlosfähigkeit des tragbaren Geräts, eine Hardwarekonnektivitätsleistung des tragbaren Geräts, eine Art von Sicherheitsfähigkeit des tragbaren Geräts, eine Empfindlichkeit eines Touchscreens des tragbaren Geräts, eine Genauigkeit des Touchscreens des tragbaren Geräts, eine Konnektivität des tragbaren Geräts mit einer Brückenvorrichtung oder eine Konnektivität des tragbaren Geräts mit einem Fernsystem ist.

14. Server nach Anspruch 12, wobei das zu testende Merkmal eine Empfindlichkeit eines Touchscreens des tragbaren Geräts, eine Genauigkeit des Touchscreens des tragbaren Geräts, eine Konnektivität des tragbaren Geräts mit einer Brückenvorrichtung oder eine Konnektivität des tragbaren Geräts mit einem Fernsystem ist.

## Revendications

1. Procédé pour autoriser un dispositif portable à communiquer avec un dispositif médical implantable, le procédé comprenant :
la réalisation d'un test qui détermine si au moins un paramètre de configuration de dispositif correspondant respectivement à au moins une caractéristique à tester sur le dispositif portable est rempli par le dispositif portable, où la au moins une caractéristique est une ou plusieurs caractéristiques sélectionnées dans le groupe constitué par :
une mémoire de stockage du dispositif portable, une mémoire à accès aléatoire du dispositif portable, une hauteur d'écran du dispositif portable, une largeur d'écran du dispositif portable, un modèle du dispositif portable, une vitesse d'unité centrale de traitement du dispositif portable, une capacité USB du dispositif portable, une capacité de communication en champ proche du dispositif portable, une capacité Bluetooth® du dispositif portable, une sensibilité d'un écran tactile du dispositif portable, et une précision de l'écran tactile du dispositif portable ;
l'autorisation du dispositif portable à communiquer avec le dispositif médical implantable uniquement si la au moins une caractéristique est remplie par le dispositif portable ; et
le refus de l'autorisation du dispositif portable à communiquer avec le dispositif médical implantable si la au moins une caractéristique n'est pas remplie par le dispositif portable.

2. Procédé selon la revendication 1, comprenant en outre la fourniture d'une interface utilisateur graphique qui permet au dispositif portable de communiquer avec le dispositif médical implantable uniquement si le dispositif portable est autorisé à communiquer avec le dispositif médical implantable.

3. Procédé selon la revendication 1, dans lequel le dispositif portable comprend un dispositif intelligent personnel.

4. Procédé selon la revendication 1, dans lequel la réalisation du test comprend la demande d'une entrée à un utilisateur, et la réception de l'entrée en provenance de l'utilisateur, où il est déterminé que la au moins une caractéristique est remplie par le dispositif portable sur la base de l'entrée reçue en provenance de l'utilisateur.

5. Procédé selon la revendication 4, dans lequel la caractéristique à tester est l'une parmi une sensibilité d'un écran tactile du dispositif portable, une précision de l'écran tactile du dispositif portable, une connectivité du dispositif portable à un dispositif de pont, et une connectivité du dispositif portable à un système distant.

6. Procédé selon la revendication 1, comprenant en outre :
la détection d'une modification dans le dispositif portable, où la modification est au moins l'une parmi une installation de logiciel ou de micrologiciel, une mise à niveau de logiciel ou de micrologiciel, une activation d'une capacité, et une désactivation d'une capacité ; et
la désautorisation automatique du dispositif portable modifié de communiquer avec le dispositif médical implantable lorsque la modification est détectée.

7. Procédé selon la revendication 6, comprenant en outre :
la réalisation du test qui détermine si le au moins un paramètre de configuration de dispositif correspondant respectivement à la au moins une caractéristique à tester est rempli par le dispositif portable modifié ; et
l'autorisation du dispositif portable modifié à communiquer avec le dispositif médical implantable si la au moins une caractéristique est remplie par le dispositif portable modifié.

8. Procédé selon la revendication 1, comprenant en outre :
la transmission, à un serveur, d'une demande pour télécharger un fichier de configuration ; et
la réception du fichier de configuration en provenance du serveur dans le dispositif portable, où le fichier de configuration inclut le au moins un paramètre de configuration de dispositif correspondant à la au moins une caractéristique à tester sur le dispositif portable.

9. Procédé selon la revendication 8, comprenant en outre :
la réception d'un fichier de configuration mis à jour en provenance du serveur, où le fichier de configuration mis à jour inclut au moins un paramètre de configuration de dispositif mis à jour correspondant à au moins une caractéristique mise à jour à tester sur le dispositif portable ; et
la désautorisation du dispositif portable lorsque le fichier de configuration mis à jour est reçu.

10. Procédé selon la revendication 9, comprenant en outre :
la réalisation d'un test qui détermine si le au moins un paramètre de configuration de dispositif correspondant respectivement à la au moins une caractéristique mise à jour à tester est rempli par le dispositif portable ; et
l'autorisation du dispositif portable à communiquer avec le dispositif médical implantable si la au moins une caractéristique mise à jour est remplie par le dispositif portable.

11. Procédé selon la revendication 1, dans lequel le au moins un paramètre de configuration de dispositif comprend une pluralité de paramètres de configuration de dispositif, la au moins une caractéristique comprend une pluralité de caractéristiques, et le dispositif portable est autorisé à communiquer avec le dispositif médical implantable uniquement si la totalité des caractéristiques sont remplies par le dispositif portable.

12. Serveur pour faciliter l'autorisation d'un dispositif portable à communiquer avec un dispositif médical implantable, dans lequel le serveur comprend :
une mémoire stockant un programme de vérification de configuration et un fichier de configuration ayant au moins un paramètre de configuration de dispositif correspondant respectivement à au moins une caractéristique à tester sur le dispositif portable, où le programme de vérification de configuration, lorsqu'il est exécuté par le dispositif portable, réalise un test qui détermine si le au moins un paramètre de configuration de dispositif est rempli par le dispositif portable, autorise le dispositif portable à communiquer avec le dispositif médical implantable uniquement si la au moins une caractéristique est remplie par le dispositif portable, et refuse l'autorisation du dispositif portable à communiquer avec le dispositif médical implantable si la au moins une caractéristique n'est pas remplie par le dispositif portable ;
un modem configuré pour communiquer à distance avec le dispositif portable ; et
un contrôleur configuré pour recevoir une demande en provenance du dispositif portable pour télécharger le fichier de configuration et le programme de vérification de configuration, et pour transmettre le fichier de configuration et le programme de vérification de configuration au dispositif portable en réponse à la demande.

13. Serveur selon la revendication 12, dans lequel la caractéristique à tester sur le dispositif portable est l'une parmi une mémoire de stockage du dispositif portable, une mémoire à accès aléatoire du dispositif portable, une capacité du dispositif portable à se connecter à un type d'un réseau, une hauteur d'écran du dispositif portable, une largeur d'écran du dispositif portable, un système d'exploitation du dispositif portable, un modèle du dispositif portable, un type d'unité centrale de traitement du dispositif portable, une vitesse d'unité centrale de traitement du dispositif portable, un type de bibliothèque graphique du dispositif portable, une capacité USB du dispositif portable, un niveau API du dispositif portable, une capacité de communication en champ proche du dispositif portable, une capacité Bluetooth® du dispositif portable, une capacité sans fil du dispositif portable, une performance de connectivité matérielle du dispositif portable, un type de capacité de sécurité du dispositif portable, une sensibilité d'un écran tactile du dispositif portable, une précision de l'écran tactile du dispositif portable, une connectivité du dispositif portable à un dispositif de pont, et une connectivité du dispositif portable à un système distant.

14. Serveur selon la revendication 12, dans lequel la caractéristique à tester est l'une parmi une sensibilité d'un écran tactile du dispositif portable, une précision de l'écran tactile du dispositif portable, une connectivité du dispositif portable à un dispositif de pont, et une connectivité du dispositif portable à un système distant.
